# EUROPEAN PATENT SPECIFICATION

(11) **EP 2 263 388 B1**
(45) Date of publication and mention of the grant of the patent: **12.02.2014**
(21) Application number: 09730486.9
(22) Date of filing: 08.04.2009
(51) Int. Cl.: A61N 1/36

(54) **ELECTRICAL STIMULATION OF THE ACOUSTIC NERVE WITH COHERENT FINE STRUCTURE**
ELEKTRISCHE STIMULATION DES HÖRNERVS MIT KOHÄRENTER FEINER STRUKTUR
STIMULATION ÉLECTRIQUE DU NERF ACOUSTIQUE AVEC UNE STRUCTURE FINE COHÉRENTE

(30) Priority: 08.04.2008 US 43170 P
(43) Date of publication of application: 22.12.2010
(73) Proprietor: Med-El Elektromedizinische Geräte GmbH, 6020 Innsbruck (AT)
(72) Inventor: ZIERHOFER, Clemens, M., A-A6250 Kundl (AT)
(74) Representative: Downing, Michael Philip
(86) International application number: PCT/US2009/039857
(87) International publication number: WO 2009/126680

(56) References cited:
- WO-A1-2009/012151
- US-A1- 2005 222 644
- US-A1- 2006 080 087
- US-A1- 2006 227 986
- US-A1- 2006 227 986
- US-A1- 2008 215 332
- US-B2- 7 139 403
- WILSON B S ET AL: "BETTER SPEECH RECOGNITION WITH COCHLEAR IMPLANTS", NATURE: INTERNATIONAL WEEKLY JOURNAL OF SCIENCE, NATURE PUBLISHING GROUP, UNITED KINGDOM, vol. 352, no. 6332, 18 July 1991 (1991-07-18), pages 236-238, XP000225858, ISSN: 0028-0836, DOI: 10.1038/352236A0

## Description

### Technical Field

The present invention relates to creating a stimulation sequence for use in electrical stimulation of the acoustic nerve, and more particularly to a coherent fine structure approach for a cochlear implant.

### Background Art

Cochlear implants are a possibility to help profoundly deaf or severely hearing impaired persons. Unlike conventional hearing aids, which just apply an amplified and modified sound signal, a cochlear implant is based on direct electrical stimulation of the acoustic nerve. The intention of a cochlear implant is to stimulate nervous structures in the inner ear electrically in such a way that hearing impressions most similar to normal hearing are obtained.

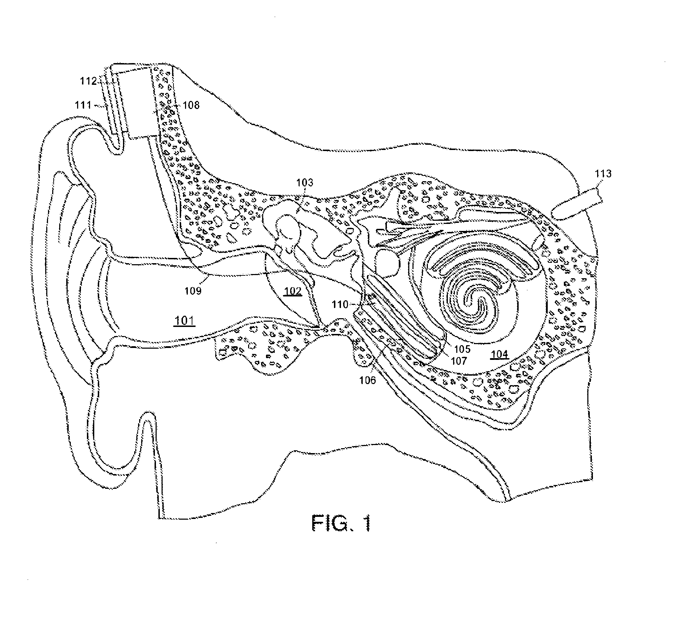

A normal ear transmits sounds as shown in Fig. 1 through the outer ear **101** to the eardrum **102,** which moves the bones of the middle ear **103,** which in turn excites the cochlea **104.** The cochlea **104** includes an upper channel known as the scala vestibuli **105** and a lower channel known as the scala tympani **106,** which are connected by the cochlear duct **107.** In response to received sounds transmitted by the middle ear **103,** the fluid filled scala vestibuli **105** and scala tympani **106** function as a transducer to transmit waves to generate electric pulses that are transmitted to the cochlear nerve **113,** and ultimately to the brain.

Cochlear implant systems have been developed to overcome this by directly stimulating the user's cochlea **104.** A cochlear implant system typically includes two parts, the speech processor and the implanted stimulator. The speech processor (not shown in Fig. 1) may include the power supply (batteries) of the overall system, and a microphone that provides an audio signal input to an external signal processing stage where various signal processing schemes can be implemented. The processed audio signal is then converted into a digital data format, such as a sequence of data frames, for transmission into receiver **108** of the implanted stimulator.

The connection between speech processor and the receiver **108** of the implanted stimulator is established either by means of a radio frequency link (transcutaneous) or by means of a plug in the skin (percutaneous). Fig. 1 shows a typical arrangement based on inductive coupling through the skin to transfer both the required electrical power and the processed audio information. As shown in Fig. 1, an external transmitter coil **111** (coupled to the external signal processor) is placed on the skin adjacent to a subcutaneous receiver coil **112** (connected to the receiver **108).** Often, a magnet in the external coil structure interacts with a corresponding magnet in the subcutaneous secondary coil structure. This arrangement inductively couples a radio frequency (rf) electrical signal to the receiver **108.** The receiver **108** is able to extract from the rf signal both the audio information for the implanted portion of the system and a power component to power the implanted system.

Besides extracting the audio information, the receiver **108** also performs additional signal processing such as error correction, pulse formation, etc., and produces a stimulation pattern (based on the extracted audio information) that is sent through connected wires **109** to an implanted electrode carrier **110.** Typically, this electrode carrier **110** includes multiple electrodes on its surface that provide selective stimulation of the cochlea **104.**

At present, the most successful stimulation strategy is the so called "continuous-interleaved-sampling strategy" (CIS) introduced by Wilson B. S., Finley C. C., Lawson D. T., Wolford R. D., Eddington D. K., Rabinowitz W. M., "Better speech recognition with cochlear implants," Nature, vol. 352, 236 - 238, July 1991B . Signal processing for CIS in the speech processor involves the following steps:
1) Splitting up of the audio frequency range into spectral bands by means of a filter bank;
2) Envelope detection of each filter output signal;
3) Instantaneous nonlinear compression of the envelope signals (map law); and,
4) Adaptation to thresholds (THR) and most comfortable loudness (MCL) levels.

According to the tonotopic organization of the cochlea, each stimulation electrode in the scala tympani is associated with a band-pass filter of the external filter bank. For stimulation, symmetrical biphasic current pulses are applied. The amplitudes of the stimulation pulses are directly obtained from the compressed envelope signals (step (3) of above). These signals are sampled sequentially, and the stimulation pulses are applied in a strictly non-overlapping sequence. Thus, as a typical CIS-feature, only one stimulation channel is active at one time.

CIS has proven to be very successful in conveying speech information, in particular for western languages such as, e.g., English, French, etc. However, some potential to improve the performance of cochlear implants can be found in the field of tonal languages such as, e.g., Mandarin, Vietnamese, etc., and in the field of music perception. In both fields, a lot of information is contained in the so called fundamental frequency, sometimes designated as the pitch frequency, and temporal variations thereof. With CIS, the fundamental frequency is only weakly represented in the temporal patterns of stimulation pulses.

US-A-2006 227 986 discloses a method of enhancing the pitch cue of an audio signal perceived by a cochlear implant recipient, wherein the audio signal is processed and input to an implant device of the recipient, the method comprising filtering the audio signal to produce a filtered audio signal, envelope detecting the filtered audio signal to produce an envelope detected signal, comparing the filtered audio signal to produce a gating signal having one of two Boolean states and multiplying the gating signal with the envelope detected signal to produce a multiplied signal.

US-A-2008 215 332 discloses a method including receiving sound containing a voiced component, extracting pitch information from said sound for the voiced component, and adding the pitch information into a continuous-interleaved-stimulation processor of a cochlear implant.

US-A-2009 018 614 discloses a cochlear implant system that includes a broad band low frequency filter associated with a stimulation electrode.

### Summary of the Invention

In accordance with the invention, there is provided a method as defined in claim 1 and a system as defined in claim 11. Methods of delivering stimulation to a patient do not form part of the invention.

### Summary of the Invention

In accordance with one embodiment of the invention, a method of enhancing temporal cues in a cochlear implant system is presented. The cochlear implant system includes an electrode array in which each electrode is stimulated based on a stimulation sequence of pulses. The method includes deriving signal c(t) from an acoustic representative electrical signal, the signal c(t) including low frequency temporal information. An estimate of spectral energy e(t) is derived from the acoustic representative electrical signal, the signal e(t) including spectral information with substantially no pitch related temporal information.

The stimulation sequence is created for at least one electrode in the array as a function of c(t) and e(t).

In accordance with related embodiments of the invention, creating the stimulation signal may include multiplying e(t) by c(t). Deriving the estimate of spectral energy e(t) includes applying the acoustic representative electrical signal to a bank of filters, each filter in the bank of filters associated with a channel that includes an electrode in the electrode array, the number of channels equal to N. The spectral energy is estimated for each channel after filtering to form eᵢ(t), (i = 1, 2, ..., N).

In accordance with further related embodiments of the invention, deriving signal c(t) may include filtering the acoustic representative electrical signal to form signal x(t), performing half wave rectification on x(t) to form signal xₕ(t), and performing amplitude normalization on xₕ(t) to form the signal c(t). Filtering may include band-pass filtering, for example, between 80 Hz to 400 Hz. Performing amplitude normalization may include performing peak detection on x(t) to form peak detector signal xₚ(t), and dividing xₕ(t) by xₚ(t) to form the signal c(t). Performing amplitude normalization may include deriving Hilbert envelope env(x(t)) of x(t), and dividing xₕ(t) by the env(x(t)) to form signal c(t). Performing amplitude normalization may include dividing xₕ(t) by x_{power}(t), wherein x_{power}(t) represents the instantaneous power of signal x(t).

In accordance with still further related embodiments of the invention, deriving signal c(t) may include filtering the acoustic representative electrical signal to form signal x(t), and associating segments x(t) > 0 to amplitude c(t) = 1, and segments x(t) < 0 to amplitude c(t) = 0.

In accordance with further embodiments of the invention, deriving signal c(t) may include using a pitch picker.

In accordance with yet further related embodiments of the invention, the method further includes applying the acoustic representative electrical signal to a bank of filters, each filter in the bank of filters associated with a channel that includes an electrode in the electrode array, the method further comprising setting c(t) equal to one for at least one channel filtered at the high frequency end. For example, c(t) may be set to one for channels covering a range higher than 1 kHz.

In accordance with another embodiment of the invention, a system for enhancing temporal cues in a cochlear implant system is presented. The cochlear implant system includes an electrode array in which each electrode is stimulated based on a stimulation sequence of pulses. A first module derives signal c(t) from an acoustic representative electrical signal, the signal c(t) including low frequency temporal information. A second module estimates spectral energy e(t) from the acoustic representative electrical signal, the signal e(t) including spectral information with substantially no pitch related temporal information. A third module creates the stimulation sequence for at least one electrode in the array as a function of c(t) and e(t).

In accordance with related embodiments of the invention, the third module may include a multiplier for multiplying c(t) and e(t). The second module includes a band of filters for filtering the acoustic representative electrical signal, each filter in the bank of filters associated with a channel that includes an electrode in the electrode array, the number of channels equal to N. An estimator estimates spectral energy for each channel after filtering to form eᵢ(t), (i = 1, 2, ..., N).

In accordance with further related embodiments of the invention, the first module includes a band-pass filter for filtering an acoustic representative electrical signal to form signal x(t). A half-wave rectifier performs half wave rectification on x(t) to form signal xₕ(t). A normalizer performs amplitude normalization on xₕ(t) to form signal c(t). The band-pass filter may pass signals between 80Hz to 400 Hz. The normalizer may include a peak detector for forming peak detector signal xₚ(t); and a divider module for dividing xₕ(t) by xₚ(t) to form the signal c(t). The normalizer may include a Hilbert module for deriving Hilbert envelope env(x(t)) of x(t), and a divider module for dividing xₕ(t) by env(x(t)) to form signal c(t). The normalizer may include a divider for dividing xₕ(t) by x_{power}(t), wherein x_{power}(t) represents the instantaneous power of signal x(t).

In accordance with yet further related embodiments of the invention, the system includes a filter for filtering the acoustic representative electrical signal, wherein the first module includes an association module for associating segments x(t) > 0 to amplitude c(t) = 1, and segments x(t) < 0 to c(t) = 0.

In accordance with further related embodiments of the invention, the first module may include a pitch picker.

In accordance with still further embodiments of the invention, the system includes a bank of filters for filtering the acoustic representative electrical signal, each filter in the bank of filters associated with a channel that includes an electrode in the electrode array, wherein the first module sets c(t) equal to one for at least one channel filtered at the high frequency end. For example, the first module may set c(t) equal to one for channels filtered at higher than 1 kHz. For example, the first module may set c(t) to one for channels covering a range higher than 1 kHz.

### Brief Description of the Drawings

The foregoing features of the invention will be more readily understood by reference to the following detailed description, taken with reference to the accompanying drawings, in which:
Fig. 1 shows elements of a typical cochlear implant system and relevant ear structures;
Figs. 2A-C show generation of carrier c(t), in accordance with various embodiments of the invention;
Figs. 3A-D show generation of a CFS signal derived from band filter no. 1 [350 Hz - 550 Hz] of a 6-channel system, in accordance with various embodiments of the invention;
Figs. 4A-C show an examplary CFS stimulation sequence and CIS stimulation sequence, derived from band filter No. 1 [350 Hz - 550 Hz] of a 6-channel system, in accordance with various embodiments of the invention. The stimulation pulse rate in both sequences is 3 kpulses/sec;
Fig. 5 shows exemplary CFS stimulation sequences of a 6-channel CFS system, assuming pulse rates of 3 kpulses/sec per channel, in accordance with various embodiments of the invention; and
Fig. 6 shows exemplary CIS stimulation sequences of a 6-channel CFS system, assuming pulse rates of 3 kpulses/sec per channel.

### Detailed Description of Specific Embodiments

In illustrative embodiments of the invention, a system and method of enhancing the representation of low frequency temporal cues associated with a cochlear implant is presented, and shall be referred to herein as the "Coherent Fine Structure (CFS)" approach. Details are discussed below.

The CFS approach is directed primarily at a better representation of temporal cues in the pitch frequency range, typically between, without limitation, 80 Hz and 400 Hz. Signal processing according to CFS may involve a filter bank, similar as for CIS. Illustratively, the overall frequency range of the audio signal may be split up by N band-pass filters, resulting in N output signals bᵢ(t) (i = 1,2, ..., N). For each filter output, an estimate of spectral energy eᵢ(t) (i = 1, 2, ..., N) is determined. According to the invention, signals eᵢ(t) are r.m.s. signals of the filter output signals. It is assumed that signals eᵢ(t) are slowly varying with time, and typically do not include frequency components higher than about the lower limit of the pitch frequency range, typically about 80 Hz.

Temporal fine structure information is introduced by a carrier signal c(t). Carrier c(t) reflects the instantaneous pitch frequency directly as temporal information. Illustratively, carrier signal c(t) may vary, for example, between amplitudes zero and one. In preferred embodiments, c(t) is multiplied with each of the estimated spectral energy signals eᵢ(t) (i = 1, 2, ..., N). The product signals c(t)eᵢ(t) are used to derive the stimulation pulse amplitudes of the individual channels of an N-channel system. Since c(t) is applied coherently for all CFS channels, the temporal structure of the pitch frequency is generally not impaired by effects due to spatial channel interaction. With the CFS concept, a clear separation between "spectral information" - represented by the envelope signals eᵢ(t) - and "fine structure information" - represented by signal c(t) - is achieved.

An example of a carrier signal c(t), and a illustrative procedure to derive carrier signal c(t) from an audio signal, is as follows:
(1) Band-pass filtering of the audio signal in the range 80 Hz to 400 Hz (resulting in signal x(t));
(2) Half wave rectification (resulting in signal xₕ(t)); and
(3) Amplitude normalization (resulting in signal c(t)).

Amplitude normalization (step (3)) may be achieved, without limitation, by utilizing a peak detector. The peak detector signal xₚ(t) tracks the positive peaks of x(t), and in between two peaks, xₚ(t) is decaying with a particular time constant τ. Typically, τ is in the range of some tens of milliseconds. Carrier c(t) is an "amplitude-normalized" version of xₕ(t), i.e., c(t) = xₕ(t)/xₚ(t). The purpose of c(t) is essentially to preserve the temporal structure of x(t).

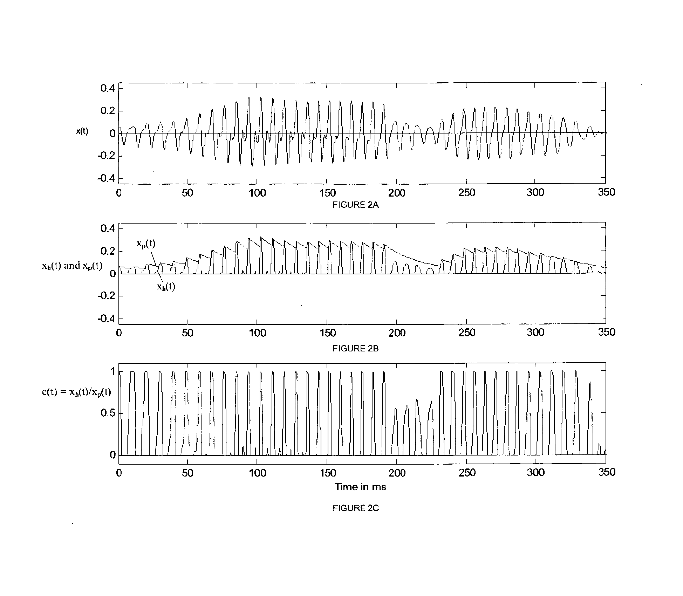

Figs. 2A-C show the generation of carrier c(t), in accordance with various embodiments of the invention. Fig. 2A shows a band-pass filtered version x(t) of a voiced speech sample. The mean amplitude is strongly varying. Fig. 2B shows the half wave rectified version xₕ(t), and the peak picker signal xₚ(t). The ratio c(t) = xₕ(t)/xₚ(t) is depicted in Fig. 2C. The amplitude of c(t) is smaller than 1, whenever amplitude reductions in x(t) occur which are faster than the time constant τ. In Fig. 2 this occurs, for example, at about t ≈ 200 to 230 ms.

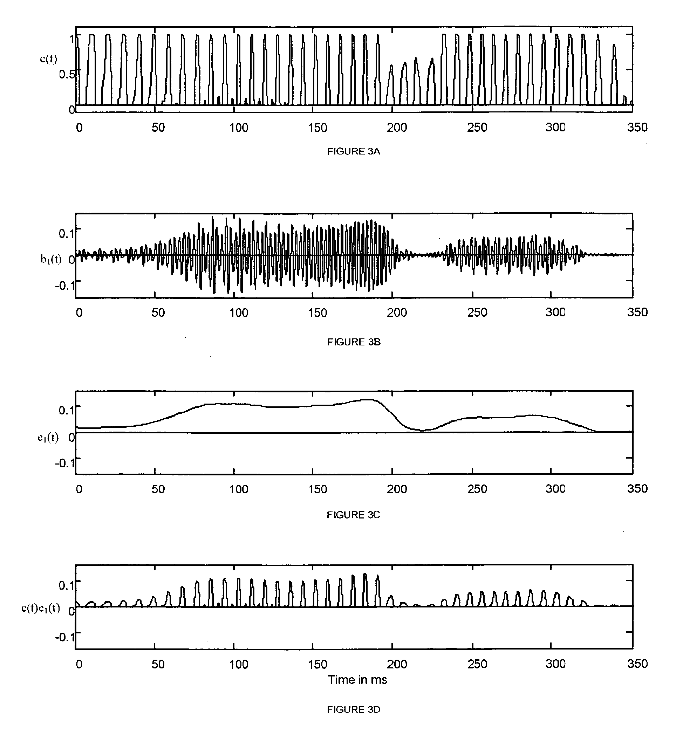

Figs. 3A-D shows an example of signals appearing in channel No. 1 of a 6-channel stimulation system, in accordance with various embodiments of the invention. More particularly, Fig. 3A depicts carrier c(t) as derived in Fig. 1. Fig. 3B depicts the band-pass output signal b₁(t) of a band-pass filter within [350 Hz - 550 Hz]. Fig. 3C depicts an estimate of spectral energy e₁(t) associated with output signal b₁(t). Obviously, e₁(t) is slowly varying with time and free of rapid temporal fluctuations. In particular, the pitch frequency is not emphasized in e₁(t). Fig. 3D depicts the product signal c(t)e₁(t). This signal now includes both the temporal fine structure of c(t) and the spectral information of e₁(t).

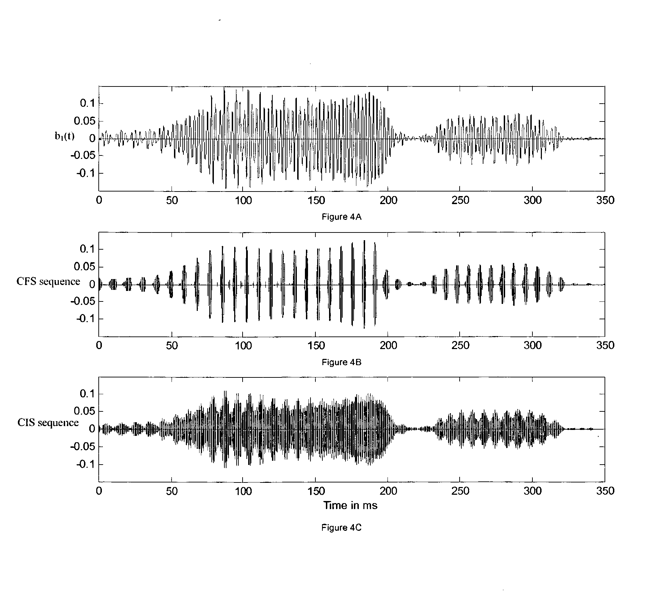

Figs. 4A-C is a comparison of the CIS and CFS approaches. Fig. 4A shows the band-pass output signal b₁(t) of a band-pass filter within [350 Hz - 550 Hz]. Fig. 4B depicts the resulting CFS sequence of stimulation pulses at a rate of 3 kpulses/sec, which is the result of sampling the CFS signal shown in Fig. 3C, in accordance with various embodiments of the invention. Each vertical line represents a biphasic stimulation pulse. The pitch frequency is clearly represented by the CFS signal in Fig. 4B. Fig. 4C depicts the resulting CIS-sequence at a rate of 3 kpulses/sec, representing the envelope of b₁(t). Obviously, the variations in the pulse amplitudes in the CIS sequence provide pitch frequency information. However, in contrast to CFS, this representation is much less pronounced.

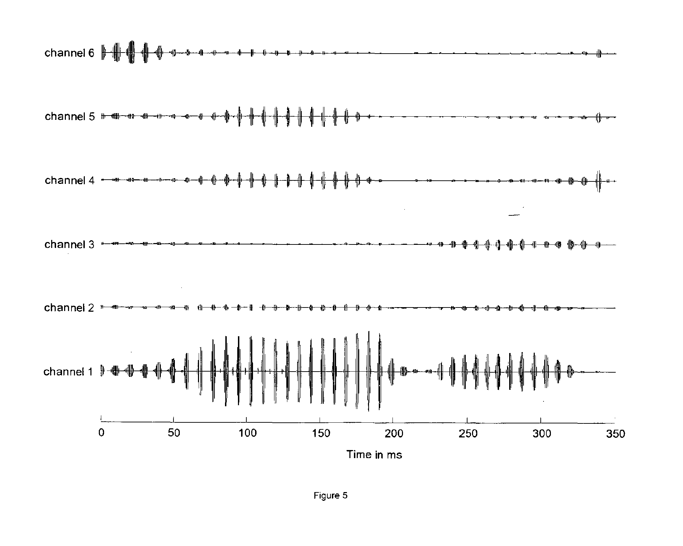

Fig. 5 shows an example of stimulation sequences of a 6-channel system with an overall frequency range is [350 Hz - 5500 Hz], in accordance with various embodiments of the invention. All channels, with channel 1 derived from the lowest band filter, and channel 6 derived from the highest band filter, are depicted. The overall pulse rate here is 18 kpulses/sec, resulting in non-overlapping pulses at repetition rates of 3 kpulses/sec for each channel. The individual stimulation sequences represent sampled versions of product signals c(t)eᵢ(t). As intended, the pitch frequency is clearly represented by the sequences of pulse bursts in the individual channels. Since the pulse bursts apply coherently in time across the channels, the pitch representation is very robust against influences due to spatial channel interaction.

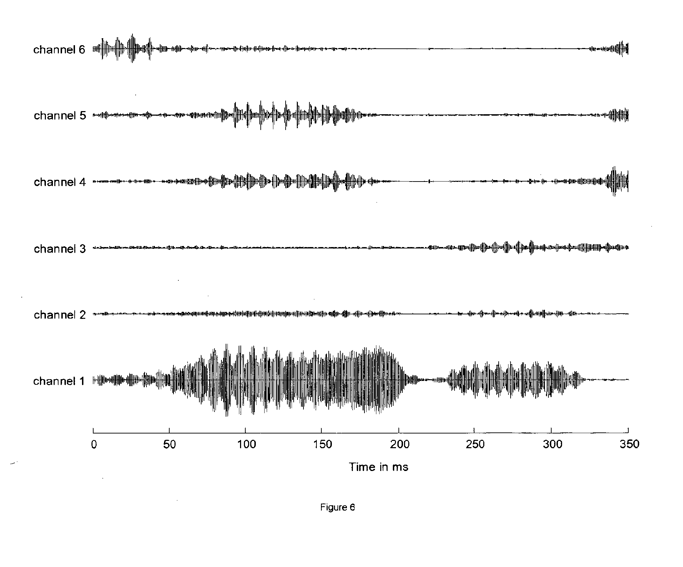

Fig. 6 presents a 6-channel CIS representation. The same voiced speech segment and the same filter bank as for the CFS representation of Fig. 4 is used. Obviously, the pitch structure is reflected by a more or less pronounced amplitude modulation. However, the overall representation of pitch is much clearer in the CFS than in the CIS representation.

The CFS concept primarily concerns audio signals where a clear pitch component is present, e.g., voiced speech segments. In various embodiments, situations without a clear pitch component may be detected, for example, by means of a voiced/unvoiced detector, and the carrier c(t) may be set to c(t) = 1. Then, product signals c(t)eᵢ(t) are equal to eᵢ(t), and hence are represented by stimulation pulses at the rate equal to the frame rate per channel.

The representation of c(t)eᵢ(t) by means of stimulation pulses can theoretically be achieved by utilizing a sufficiently high pulse repetition rate, However, if the overall pulse repetition rate for an adequate temporal resolution of signals c(t)eᵢ(t) would be too high, supporting concepts such as: "Channel Interaction Compensation (CIC)" (for simultaneous stimulation) as described in Zierhofer C.M., "Electrical nerve stimulation based on channel specific sampling sequences," U.S. Patent No. 6,594,525, 2003; and/or the "Selected Group (SG)" algorithm, as described in Zierhofer C.M., " Electrical stimulation of the acoustic nerve based on selected groups," U.S. Patent Application 20050203589 (pending) can be utilized. Note that while the CSSS approach as described in U.S. Patent No. 6,594,525 clearly enhances the temporal fine structure in the individual channels, the fine structure is not presented coherently.

In practical applications, the low frequency information may be removed for channels at the high frequency end, by setting c(t) equal to one. This prevents low frequency temporal information from being in conflict with the frequency which is associated with the electrode position (tonotopic principle). For example, c(t) may be set to one for channels covering a range higher than 1 kHz. For these particular channels, the stimulation is similar to CIS.

In various embodiments, carrier c(t) may be obtained by xₕ(t)/env(x(t)), where xₕ(t) is the half wave rectified version of the band-pass filtered audio signal x(t), and env(x(t)) is its Hilbert envelope. Still another method to obtain carrier c(t) may be to simply associate segments x(t) > 0 to amplitude c(t) = 1, and segments x(t) < 0 to c(t) = 0. In this case, only the zero crossings of x(t) are used to encode the temporal fine structure. Still yet another method to obtain carrier c(t) may be to compute c(t) = xₕ(t)/x_{power}(t), where x_{power}(t) is an estimate of the instantaneous power of signal x(t).

Another method to obtain a carrier signal c(t) may be based on a pitch picker. Examples of pitch pickers are described, without limitation, in W. Hess, "Pitch determination of speech signals," Ed. Springer, Berlin, 1983.

In various embodiments, the band-pass filtered version x(t) of the audio signal may cover the range of about [100 Hz - 1000 Hz], covering the frequency ranges pitch- and first formant frequency.

In various embodiments, the disclosed method may be implemented as a computer program product for use with a computer system. Such implementation may include a series of computer instructions fixed either on a tangible medium, such as a computer readable media (*e.g.,* a diskette, CD-ROM, ROM, or fixed disk) or transmittable to a computer system, via a modem or other interface device, such as a communications adapter connected to a network over a medium. Medium may be either a tangible medium (*e.g.,* optical or analog communications lines) or a medium implemented with wireless techniques (*e.g.,* microwave, infrared or other transmission techniques). The series of computer instructions embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable media with accompanying printed or electronic documentation (*e.g.,* shrink wrapped software), preloaded with a computer system (*e.g.,* on system ROM or fixed disk), or distributed from a server or electronic bulletin board over the network (*e.g*., the Internet or World Wide Web).

The embodiments of the invention described above are intended to be merely exemplary; numerous variations and modifications will be apparent to those skilled in the art. All such variations and modifications are intended to be within the scope of the present invention as defined in any appended claims.

## Claims

1. A method of enhancing temporal cues in a cochlear implant system, the cochlear implant system including an electrode array ; the method comprising:
applying an acoustic representative electrical signal to a bank of filters, each filter in the bank of filters associated with a channel that includes an electrode in the electrode array, the number of channels equal to N;
deriving signal c(t) from the acoustic representative electrical signal, the signal c(t) including low frequency temporal information;
deriving an estimate of spectral energy e(t) for each channel after filtering to form eᵢ(t), (i = 1, 2, ..., N) including spectral information with substantially no pitch related temporal information, wherein eᵢ(t) is a r.m.s. signal based on its associated filter output; and
creating a stimulation sequence for a plurality of the electrodes in the array as a function of c(t) and eᵢ(t).

2. The method according to claim 1, wherein creating the stimulation sequence includes multiplying eᵢ(t) by c(t).

3. The method according to claim 1, wherein deriving signal c(t) includes:
band-pass filtering the acoustic representative electrical signal to form signal x(t); performing half wave rectification on x(t) to form signal xₕ(t); and performing amplitude normalization on xₕ(t) to form the signal c(t).

4. The method according to claim 3, wherein band-pass filtering includes passing signals between 80Hz to 400 Hz.

5. The method according to claim 3, wherein performing amplitude normalization includes:
performing peak detection on x(t) to form peak detector signal xₚ(t); and
dividing xₕ(t) by xₚ(t) to form the signal c(t).

6. The method according to claim 3, wherein performing amplitude normalisation includes:
deriving Hilbert envelope env(x(t)) of x(t); and
dividing xₕ(t) by the env(x(t)) to form signal c(t).

7. The method according to claim 3, wherein performing amplitude normalization includes dividing x_{b}(t) by x_{power}(t), wherein x_{power}(t) represents the instantaneous power of signal x(t).

8. The method according to claim 1, wherein deriving signal c(t) includes:
filtering the acoustic representative electrical signal to form signal x(t); and
associating segments x(t) > 0 to amplitude c(t) = 1, and segments x(t) < 0 to amplitude c(t) = 0.

9. The method according to claim 1, wherein deriving signal c(t) includes a pitch picker.

10. The method according to claim 1, further including:
setting c(t) equal to one for at least one channel filtered at the high frequency end.

11. A system for enhancing temporal cues in a cochlear implant system, the cochlear implant system including:
an electrode array in which each electrode can be stimulated based on a stimulation sequence of pulses;
a bank of filters for filtering an acoustic representative electrical signal, each filter in the bank of filters associated with a channel that includes an electrode in the electrode array, the number of channels equal to N;
a first module for deriving signal c(t) from the acoustic representative electrical signal, the signal c(t) including low frequency temporal information;
a second module for estimating spectral energy e(t) for each channel after filtering to form eᵢ(t), (i = 1, 2, ..., N), the signal eᵢ(t) including spectral information with substantially no pitch related temporal information, wherein eᵢ(t) is a r.m.s. signal based on its associated filter output; and
a third module for creating the stimulation sequence for a plurality of the electrodes in the array as a function of c(t) and eᵢ(t).

12. The system according to claim 11, wherein the third module for creating the stimulation sequence includes a multiplier for multiplying eᵢ(t) by c(t).

13. The system according to claim 11, wherein the first module includes:
a band-pass filter for filtering an acoustic representative electrical signal to form signal x(t);
a half-wave rectifier for performing half wave rectification on x(t) to form signal xₕ(t);
a normalizer for performing amplitude normalization on xₕ(t) to form signal c(t).

14. The system according to claim 13, wherein the band-pass filter passes signals between 80Hz to 400 Hz.

15. The system according to claim 13, wherein the normalizer includes a peak detector for forming peak detector signal xₚ(t); and a divider module for dividing xₕ(t) by xₚ(t) to form the signal c(t).

16. The system according to claim 13, wherein the normalizer includes a Hilbert module for deriving Hilbert envelope env(x(t)) of x(t), and a divider module for dividing xₕ(t) by env(x(t)) to form signal c(t).

17. The system according to claim 13, wherein the normalizer includes a divider for dividing xₕ(t) by x_{power}(t), wherein x_{power}(t) represents the instantaneous power of signal x(t).

18. The system according to claim 11, further comprising a filter for filtering the acoustic representative electrical signal, wherein the first module includes an association module for associating segments x(t) > 0 to amplitude c(t) = 1, and segments x(t) < 0 to amplitude c(t) = 0.

19. The system according to claim 11, wherein the first module includes a pitch picker.

20. The system according to claim 11, wherein the first module sets c(t) equal to one for at least one channel filtered at the high frequency end.

## Patentansprüche

1. Verfahren zum Verstärken temporaler Reize in einem Schneckenimplantatsystem, wobei das Schneckenimplantatsystem eine Elektrodenanordnung umfasst, wobei das Verfahren Folgendes umfasst:
Anlegen eines akustischen repräsentativen elektrischen Signals an eine Filterbank, wobei jeder Filter in der Filterbank mit einem Kanal verknüpft ist, der eine Elektrode in der Elektrodenanordnung umfasst, wobei die Anzahl der Kanäle gleich N ist;
Ableiten des Signals c(t) aus dem akustischen repräsentativen elektrischen Signal, wobei das Signal c(t) niederfrequente temporale Informationen umfasst;
Ableiten einer Schätzung von Spektralenergie e(t) für jeden Kanal nach dem Filtern, um eᵢ(t), (i = 1, 2, ... , N), zu erhalten, das Spektralinformationen im Wesentlichen ohne Tonhöhen-bezogene temporale Informationen enthält, wobei eᵢ(t) ein effektives Signal (r.m.s.) ist, das auf seinem zugeordneten Filterausgang basiert; und
Erzeugen einer Stimulationssequenz für mehrere der Elektroden in der Anordnung als eine Funktion von c(t) und eᵢ(t).

2. Verfahren nach Anspruch 1, wobei das Erzeugen der Stimulationssequenz das Multiplizieren von eᵢ(t) mit c(t) umfasst.

3. Verfahren nach Anspruch 1, wobei das Ableiten des Signals c(t) Folgendes umfasst:
Bandpassfiltern des akustischen repräsentativen elektrischen Signals, um das x(t) zu bilden,
Ausführen einer Halbwellengleichrichtung an x(t), um ein Signal xₕ(t) zu bilden, und
Ausführen einer Amplitudennormalisierung an xₕ(t), um das Signal c(t) zu bilden.

4. Verfahren nach Anspruch 3, wobei das Bandpassfiltern das Durchlassen von Signalen zwischen 80 Hz und 440 Hz umfasst.

5. Verfahren nach Anspruch 3, wobei das Ausführen einer Amplitudennormalisierung Folgendes umfasst:
Ausführen einer Spitzendetektion an x(t), um ein Spitzendetektorsignal xₚ(t) zu bilden; und
Teilen von xₕ(t) durch xₚ(t), um das Signal c(t) zu bilden.

6. Verfahren nach Anspruch 3, wobei das Ausführen einer Amplitudennormalisierung Folgendes umfasst:
Ableiten der Hilbert-Hüllkurve env(x(t)) von x(t); und
Teilen von xₕ(t) durch die env(x(t)), um das Signal c(t) zu bilden.

7. Verfahren nach Anspruch 3, wobei das Ausführen einer Amplitudennormalisierung das Teilen von xₕ(t) durch x_{power}(t) umfasst, wobei x_{power}(t) die Augenblicksleistung von Signal x(t) darstellt.

8. Verfahren nach Anspruch 1, wobei das Ableiten des Signals c(t) Folgendes umfasst:
Filtern des akustischen repräsentativen elektrischen Signals, um das Signal x(t) zu bilden; und
Verknüpfen von Segmenten x(t) > 0 mit der Amplitude c(t) = 1 und von Segmenten x(t) < 0 mit der Amplitude c(t) = 0.

9. Verfahren nach Anspruch 1, wobei das Ableiten des Signals c(t) einen Tonhöhensensor umfasst.

10. Verfahren nach Anspruch 1, das des Weiteren Folgendes umfasst:
Einstellen von c(t) gleich Eins für mindestens einen Kanal, der am hochfrequenten Ende gefiltert wird.

11. System zum Verstärken temporaler Reize in einem Schneckenimplantatsystem, wobei das Schneckenimplantatsystem Folgendes umfasst:
eine Elektrodenanordnung, wobei jede Elektrode auf der Grundlage einer Stimulationssequenz aus Impulsen stimuliert werden kann;
eine Filterbank zum Filtern eines akustischen repräsentativen elektrischen Signals, wobei jeder Filter in der Filterbank mit einem Kanal verknüpft ist, der eine Elektrode in der Elektrodenanordnung umfasst, wobei die Anzahl der Kanäle gleich N ist
ein erstes Modul zum Ableiten des Signals c(t) aus dem akustischen repräsentativen elektrischen Signal, wobei das Signal c(t) niederfrequente temporale Informationen umfasst;
ein zweites Modul zum Schätzen von Spektralenergie e(t) für jeden Kanal nach dem Filtern, um eᵢ(t), (i = 1, 2, ... , N), zu erhalten, wobei das Signal eᵢ(t) Spektralinformationen im Wesentlichen ohne Tonhöhen-bezogene temporale Informationen umfasst, wobei eᵢ(t) ein effektives Signal (r.m.s.) ist, das auf seinem zugeordneten Filterausgang basiert; und
ein drittes Modul zum Erzeugen der Stimulationssequenz für mehrere der Elektroden in der Anordnung als eine Funktion von c(t) und eᵢ(t).

12. System nach Anspruch 11, wobei das dritte Modul zum Erzeugen der Stimulationssequenz einen Multiplikator zum Multiplizieren von eᵢ(t) mit c(t) umfasst.

13. System nach Anspruch 11, wobei das erste Modul Folgendes umfasst:
ein Bandpassfilter zum Filtern eines akustischen repräsentativen elektrischen Signals, um das Signal x(t) zu bilden;
einen Halbwellengleichrichter zum Ausführen einer Halbwellengleichrichtung an x(t), um das Signal xₕ(t) zu bilden;
einen Normalisierer zum Ausführen einer Amplitudennormalisierung an xₕ(t), um das Signal c(t) zu bilden.

14. System nach Anspruch 13, wobei das Bandpassfilter Signale zwischen 80 Hz und 400 Hz durchlässt.

15. System nach Anspruch 13, wobei der Normalisierer einen Spitzendetektor zum Bilden eines Spitzendetektorsignals xₚ(t) sowie ein Teilermodul zum Teilen von xₕ(t) durch xₚ(t) zum Bilden des Signals c(t) umfasst.

16. System nach Anspruch 13, wobei der Normalisierer ein Hilbert-Modul zum Ableiten einer Hilbert-Hüllkurve env(x(t)) von x(t) und ein Teilermodul zum Teilen von xₕ(t) durch env(x(t)) zum Bilden des Signals c(t) umfasst.

17. System nach Anspruch 13, wobei der Normalisierer einen Teiler zum Teilen von xₕ(t) durch x_{power}(t) umfasst, wobei x_{power}(t) die Augenblicksleistung des Signals x(t) darstellt.

18. System nach Anspruch 11, das des Weiteren einen Filter zum Filtern des akustischen repräsentativen elektrischen Signals umfasst, wobei das erste Modul ein Verknüpfungsmodul zum Verknüpfen von Segmenten x(t) > 0 mit der Amplitude c(t) =1 und von Segmenten x(t) < 0 mit der Amplitude c(t) = 0 umfasst.

19. System nach Anspruch 11, wobei das erste Modul einen Tonhöhensensor umfasst.

20. System nach Anspruch 11, wobei das erste Modul c(t) gleich Eins für mindestens einen Kanal, der am hochfrequenten Ende gefiltert wird, einstellt.

## Revendications

1. Procédé d'amélioration de repères temporels dans un système d'implant cochléaire, le système d'implant cochléaire incluant un ensemble d'électrodes ; le procédé comprenant :
l'application d'un signal électrique acoustique représentatif à un banc de filtres, chaque filtre dans le banc de filtres associé avec un canal qui inclut une électrode dans l'ensemble d'électrodes, le nombre de canaux égal à N;
la dérivation d'un signal c(t) à partir du signal électrique acoustique représentatif, le signal c(t) incluant une information temporelle basse fréquence ;
la dérivation d'une estimation d'une énergie spectrale e(t) pour chaque canal après filtrage pour former eᵢ(t) (i = 1, 2, ..., N), incluant une information spectrale avec sensiblement pas d'information temporelle se rapportant à une tonie, dans lequel eᵢ(t) est un signal effectif (r.m.s). basé sur sa sortie de filtre associé ; et
la création d'une séquence de stimulation pour une pluralité des électrodes dans l'ensemble comme une fonction de c(t) et eᵢ(t).

2. Procédé selon la revendication 1, dans lequel la création de la séquence de stimulation inclut la multiplication de eᵢ(t) par c(t).

3. Procédé selon la revendication 1, dans lequel la dérivation du signal c(t) inclut :
le filtrage passe-bande du signal électrique acoustique représentatif pour former un signal x(t);
l'exécution d'un redressement de demi-onde sur x(t) pour former un signal xₕ(t); et
l'exécution d'une normalisation d'amplitude sur xₕ(t) pour former le signal c(t).

4. Procédé selon la revendication 3, dans lequel le filtrage passe-bande inclut le passage de signaux entre 80 Hz à 400 Hz.

5. Procédé selon la revendication 3, dans lequel l'exécution de la normalisation d'amplitude inclut:
l'exécution d'une détection de pic sur x(t) pour former un signal xₚ(t) de détecteur de pic ; et
la division de xₕ(t) par xₚ(t) pour former le signal c(t).

6. Procédé selon la revendication 3, dans lequel l'exécution de la normalisation d'amplitude inclut :
la dérivation d'une enveloppe de Hilbert env(x(t)) de x(t) ; et
la division de xₕ(t) par env(x(t)) pour former le signal c(t).

7. Procédé selon la revendication 3, dans lequel l'exécution de la normalisation d'amplitude inclut la division de xₕ(t) par x_{power}(t), dans lequel x_{power}(t) représente la puissance instantanée du signal x(t).

8. Procédé selon la revendication 1, dans lequel la dérivation du signal c(t) inclut :
le filtrage du signal électrique acoustique représentatif pour former le signal x(t) ; et
l'association de segments x(t) > 0 à une amplitude c(t) = 1, et de segments x(t) < 0 à une amplitude c(t) = 0.

9. Procédé selon la revendication 1, dans lequel la dérivation du signal c(t) inclut un capteur de tonie.

10. Procédé selon la revendication 1, incluant en outre :
la fixation de c(t) égal à un pour au moins un canal filtré à l'extrémité de fréquence élevée.

11. Système pour améliorer des repères temporels dans un système d'implant cochléaire, le système d'implant cochléaire incluant :
un ensemble d'électrodes dans lequel chaque électrode peut être stimulée sur la base d'une séquence d'impulsions de stimulation ;
un banc de filtres pour filtrer un signal électrique acoustique représentatif, chaque filtre dans le banc de filtres associé avec un canal qui inclut une électrode dans l'ensemble d'électrodes, le nombre de canaux égal à N;
un premier module pour dériver un signal c(t) à partir du signal électrique acoustique représentatif, le signal c(t) incluant une information temporelle basse fréquence ;
un deuxième module pour estimer une énergie spectrale e(t) pour chaque canal après filtrage pour former eᵢ(t) (i = 1, 2, ..., N), le signal eᵢ(t) incluant une information spectrale avec sensiblement pas d'information temporelle se rapportant à une tonie, dans lequel eᵢ(t) est un signal effectif (r.m.s). basé sur sa sortie de filtre associé ; et
un troisième module pour créer la séquence de stimulation pour une pluralité des électrodes dans l'ensemble comme une fonction de c(t) et eᵢ(t).

12. Système selon la revendication 11, dans lequel le troisième module pour créer la séquence de stimulation inclut un multiplicateur pour multiplier eᵢ(t) par c(t).

13. Système selon la revendication 11, dans lequel le premier module inclut :
un filtre passe-bande pour filtrer un signal électrique acoustique représentatif pour former un signal x(t) ;
un redresseur de demi-onde pour exécuter un redressement de demi-onde sur x(t) pour former un signal xₕ(t) ;
un normaliseur pour exécuter une normalisation d'amplitude sur xₕ(t) pour former le signal c(t).

14. Système selon la revendication 13, dans lequel le filtre passe-bande laisse passer des signaux entre 80 Hz à 400 Hz.

15. Système selon la revendication 13, dans lequel le normaliseur inclut un détecteur de pic pour former un signal xₚ(t) de détecteur de pic ; et un module diviseur pour diviser xₕ(t) par xₚ(t) pour former le signal c(t).

16. Système selon la revendication 13, dans lequel le normaliseur inclut un module de Hilbert pour dériver une enveloppe de Hilbert env(x(t)) de x(t), un module diviseur pour diviser xₕ(t) par env(x(t)) pour former le signal c(t).

17. Système selon la revendication 13, dans lequel le normaliseur inclut un diviseur pour diviser xₕ(t) par x_{power}(t), dans lequel x_{power}(t) représente la puissance instantanée du signal x(t).

18. Système selon la revendication 11, comprenant en outre un filtre pour filtrer le signal électrique acoustique représentatif, dans lequel le premier module inclut un module d'association pour associer des segments x(t) > 0 à une amplitude c(t) = 1, et des segments x(t) < 0 à une amplitude c(t) = 0.

19. Système selon la revendication 11, dans lequel le premier module inclut un capteur de tonie.

20. Système selon la revendication 11, dans lequel le premier module fixe c(t) égal à un pour au moins un canal filtré à l'extrémité de fréquence élevée.
